# EUROPÄISCHE PATENTSCHRIFT

(11) **EP 2 063 948 B1**
(45) Veröffentlichungstag und Bekanntmachung des Hinweises auf die Patenterteilung: **05.05.2010**
(21) Anmeldenummer: 07819490.9
(22) Anmeldetag: 31.10.2007
(51) Int. Cl.: A61M 25/01

(54) **KATHETER**
CATHETER
CATHÉTER

(30) Priorität: 03.11.2006 DE 102006051978
(43) Veröffentlichungstag der Anmeldung: 03.06.2009
(73) Patentinhaber: Smiths Medical Deutschland GmbH, 85614 Kirchseeon (DE)
(72) Erfinder: WEBER, Jörg, 83533 Edling (DE); BECK, Bernd, 72414 Rangendingen (DE)
(74) Vertreter: Schenk, Markus
(86) Internationale Anmeldenummer: PCT/EP2007/009458
(87) Internationale Veröffentlichungsnummer: WO 2008/052764

(56) Entgegenhaltungen:
- WO-A-97/29684
- WO-A-99/48424
- US-A- 4 577 637
- US-A- 5 766 151

## Beschreibung

Die vorliegende Erfindung bezieht sich auf Katheter und das Legen derselben im allgemeinen und in Ausführungsbeispielen auf Venenkatheter und insbesondere zentrale Venenkatheter, wie z.B. in Form von Mehrlumenkathetern.

Ein Venenkatheter oder auch zentraler Venenkatheter bezeichnet einen flexiblen Schlauch mit einem oder mehreren Kanälen bzw. Lumen in demselben, der in das Venensystem eingeführt wird, bis dessen distales Ende kurz vor dem Herzen liegt, und so, dass es durch die Lumenöffnungen möglich ist, Messungen durchzuführen, wie z.B. des zentralvenösen Druckes, bzw. Flüssigkeit zuzuführen, wie z.B. Elektrolyt- und Nährstofflösungen oder Medikamente. Ein Einlumenkatheter besitzt nur ein Lumen, dessen Öffnung sich beispielsweise am distalen Ende des Katheters befindet. Bei Mehrlumenkathetern können die Lumenöffnungen entlang der Länge des Katheters in Abständen angeordnet sein, um zu verhindern, dass sich die über die Lumen zu verabreichenden Reagenzien gegenseitig beeinflussen. Das Einführen bzw. die Katheterisierung des Venenkatheters erfolgt meistens über eine zentrale Vene mit der Seldinger-Technik, bei der zunächst über eine Nadel ein Führungsdraht an die Zielposition gelegt wird. Über den Führungsdraht wird der Katheter vorgeschoben und bis zu einer gewünschten Position geführt, um dort zu enden. Die Navigation bzw. die Lagekontrolle des Drahtes kann zum Beispiel über eine EKG-Ableitung erfolgen, und zwar unter Ausnutzung der elektrischen Impulse, die die Herzschläge auslösen, und insbesondere unter Ausnutzung der Polarisationsumkehr in dem Fall des Vorbeigleitens des Führungsdrahtes am Sinusknoten. Sobald der Führungsdraht die gewünschte Position erreicht hat, wird der Venenkatheter über den Führungsdraht vorgeschoben, bis sein proximales Ende mit einer entsprechenden Markierung an dem Führungsdraht übereinstimmt, woraufhin der Führungsdraht wieder entfernt wird. Alternativ oder zusätzlich zur EKG-Lagekontrolle des Venenkatheters kann die Lagekontrolle mittels Röntgendurchleuchtung stattfinden, wozu das Material des flexiblen Venenkatheters beispielsweise einen hohen Bestandteil aus Bariumsulfat aufweist.

Nachteilhaft an der zuvor skizzierten Vorgehensweise ist ein relativ hoher Aufwand zur Navigation bzw. Lagekontrolle des distalen Endes des Venenkatheters, da die EKG-Ableitung nicht immer zuverlässige Ergebnisse liefert oder nur eingeschränkt eingesetzt werden kann und die Röntgenkontrolle mit einem hohen Kosten- und Zeitaufwand verbunden ist.

Die WO 97/29684 A1 beschreibt einen Katheter mit einem Lumen, der an seiner Spitze mit einem Positionsdetektor ausgestattet ist. Der Positionssensor könne auch in einem Bauteil eingebaut sein, das dazu angepasst ist, in ein Lumen eines Katheters eingefügt zu werden, wobei im Betrieb dann der Positionssensor in das Lumen eingefügt werden würde, so dass der Katheter zu einer bestimmten Position geführt werden könnte. Daraufhin würde der externe Katheter an der Position fixiert, der Positionssensor wieder entfernt und ein anderes Werkzeug in das Lumen eingefügt. Es wird beschrieben, dass der Positionssensor auch ein Empfänger für ein akustisches Feld bzw. für Ultraschallsignale oder ein Sender für selbige Signale sein könne.

Die US 5,766,151 beschreibt, dass es gegenüber der fluoroskopischen Abbildung vorteilhaft sei, eine Ultraschallabbildung zu verwenden, um die Position einer Katheterspitze innerhalb der Aorta zu ermöglichen. Als Ultraschallmarkierungen werden luftgefüllte Taschen, Schaum oder dergleichen vorgeschlagen.

Die US 4,577,637 beschreibt einen Mehrlumenkatheter mit einem Lumen, das sich bis zur Spitze des Katheters erstreckt, und Lumen, die an der Spitze enden. Der Katheter wird durch Extrudieren gebildet, nach welchem die Spitze geformt wird, die die Lumen verschließt und nur das Lumen hindurchlässt, woraufhin Seitenaugen für die Lumen gebildet werden. In den "toten"-Abschnitten der Lumen sind Stopfen angeordnet, die aus spiralförmigen Metallfedern mit runden Enden bestehen. Sie werden dort angeordnet, bevor die Lumen durch Formung der Spitze verschlossen werden. Es wird beschrieben, dass die Federn aus Stahl, Platin oder Gold sein könnten, und dass die Stopfen dort in dem toten Abschnitt der jeweiligen Lumen sein könnten, wie z.B. durch ein Cyanoacrylat-Haftmittel. Die Stopfen 26 und 28 sind auf diese Weise vollständig eingekapselt.

Die WO 99/48424 A1 beschreibt eine Vorrichtung zur Verwendung beim Platzieren einer nicht-sterilen Sensorsonde, wie z.B. eines Ultraschallscan-Sende/Empfängers, an einer gewünschten Position innerhalb eines Patientenkörpers. Die Vorrichtung weist eine Röhre zur Aufnahme der Sonde auf, wobei die Röhre an ihrem distalen Ende geschlossen ist, so dass die Sonde, die in die Röhre durch ein proximales Ende derselben gestickt wird, von einem Kontakt mit oder einer Kontaminierung des Inneren des Patientenkörpers abgehalten wird.

Die Aufgabe der vorliegenden Erfindung besteht deshalb darin, einen Katheter sowie ein Verfahren zum Vorbereiten eines Katheters für ein Legen desselben zu schaffen, so dass ein zuverlässigeres und/oder weniger aufwendiges Legen eines Katheters möglich ist.

Diese Aufgabe wird durch einen Katheter gemäß Anspruch 1 und ein Verfahren zum Vorbereiten eines Katheters für ein Legen desselben gemäß Anspruch 6 gelöst.

Ein Kerngedanke der vorliegenden Erfindung besteht darin, dass mit verhältnismäßig wenig Aufwand ein Katheter in Ultraschallbildern leicht sichtbar gemacht werden kann und somit eine Lagekontrolle eines Katheters mit verhältnismäßig wenig Aufwand ermöglicht werden kann, indem an dem distalen Ende eines Katheters eine in einem Körper eines Lebewesens mittels Ultraschall erkennbare Ultraschallmarkierungseinrichtung vorgesehen wird, und zwar beispielsweise vorübergehend, um im Anschluss an eine erfolgreiche Heranführung des distalen Endes des Katheters an eine Sollposition der Ultraschallmarkierungseinrichtung wieder entfernt zu werden.

Gemäß einem Ausführungsbeispiel ist es vorgesehen, eine längliche passive oder aktive Sonde zur vorübergehenden Verwendung während der Katheternavigation bzw. Lagekontrolle und anschließenden Entfernung in ein Lumen eines Katheters einzuführen und in dieser Form beispielsweise als Kathetersystem zu verpacken und zu sterilisieren.

Bevorzugte Ausführungsbeispiele der vorliegenden Erfindung werden nachfolgend bezugnehmend auf die beiliegenden Zeichnungen näher erläutert. Es zeigt:
- Fig. 1: eine schematische Zeichnung eines Zwei-Lumen-Katheters; und
- Fig. 2: eine schematische Schnittansicht des distalen Endes des Zwei-Lumen-Katheters von Fig. 1 gemäß einem Vergleichsbeispiel;
- Fig. 3: eine schematische Zeichnung einer Schnittansicht des distalen Endes des Zwei-Lumen-Katheters von Fig. 1 gemäß einem weiteren Vergleichsbeispiel;
- Fig. 4: eine Schnittansicht des Zwei-Lumen-Katheters von Fig. 2 bzw. Fig. 3 entlang der Schnittlinie A-A;
- Fig. 5: eine schematische Schnittansicht eines distalen Endes eines Einlumenkatheters mit eingeführter passiver Sonde eines Kathetersystems gemäß einem Ausführungsbeispiel der vorliegenden Erfindung; und
- Fig. 6: eine schematische Zeichnung des proximalen Endes der Anordnung von Fig. 5.

Bevor im Folgenden bezugnehmend auf die Zeichnungen Ausführungsbeispiele der vorliegenden Erfindung näher erläutert werden, wird darauf hingewiesen, dass gleiche Elemente in diesen Figuren mit gleichen Bezugszeichen versehen sind, und dass eine wiederholte Beschreibung dieser Elemente weggelassen wird.

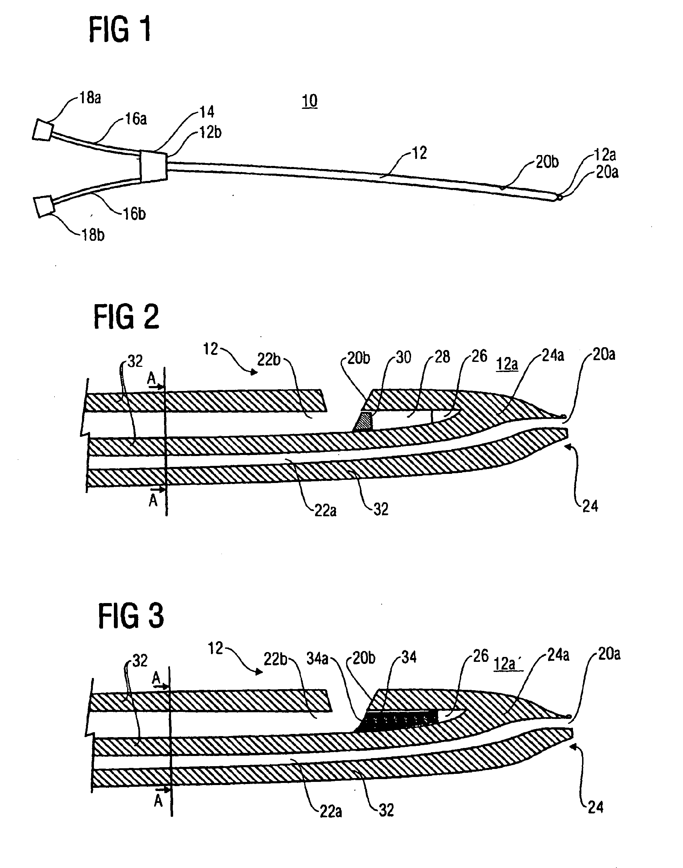

Fig. 1 zeigt exemplarisch ein Zwei-Lumen-Katheter, der beispielsweise als Venenkatheter eingesetzt werden kann. Der Katheter von Fig. 1, der allgemein mit 10 angezeigt ist, umfasst einen flexiblen Katheterschlauch 12 mit einem distalen Ende 12a und einem proximalen Ende 12b, einen Verteiler 14, zwei Anschlussschläuche 16a und 16b und zwei Steckanschlüsse 18 und 18b, die als Schraubkonnektoren ausgeführt sein können und im Folgenden auch manchmal als Luerlockanschlüsse bezeichnet werden. Der Katheterschlauch 12 weist im Inneren (in Fig. 1 nicht sichtbar) zwei Kanäle bzw. Lumen auf, von denen sich das eine an einer Lumenöffnung 20a öffnet, die an der distalen Spitze angeordnet ist, und das andere Lumen sich an einer seitlichen Öffnung 20b öffnet, die mit einem Abstand von der distalen Spitze bzw. proximaler aber immer noch in der Nähe des distalen Endes 12a angeordnet ist. Der Verteiler 14 verbindet an dem proximalen Ende 12b eines der Lumen mit dem Anschlussschlauch 16a und das andere Lumen mit dem Anschlussschlauch 16b, die wiederum mit jeweils einem der Luerlockanschlüsse 18a, 18b verbunden sind, so dass an die selben weitere Schläuche zur Flüssigkeitszufuhr oder Blutentnahme oder sonstigen fluidmäßigen Kopplung mit den Lumenöffnungen 20a, 20b anschließbar sind.

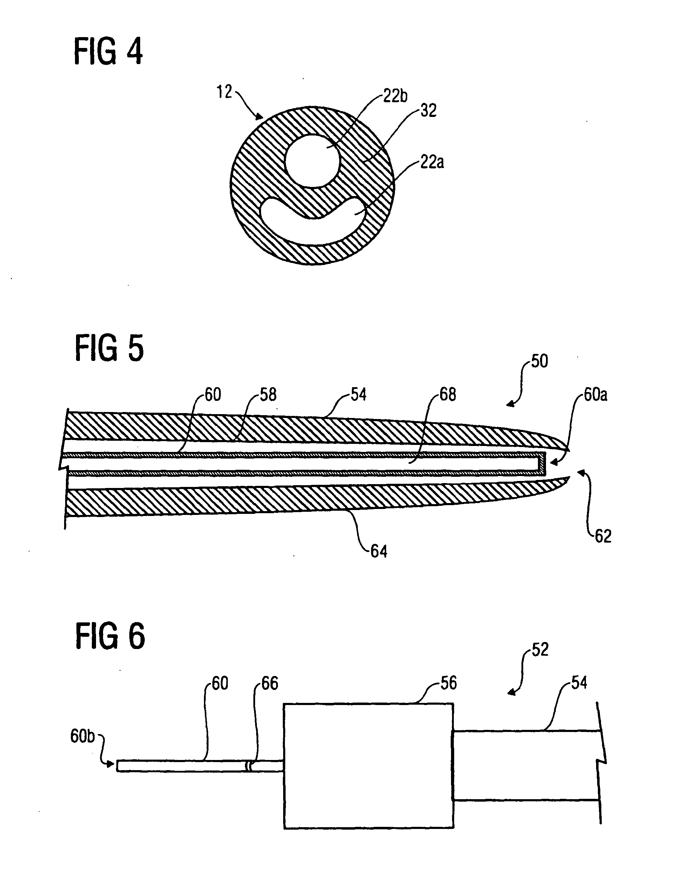

Fig. 2, welche nicht Bestandteil der vorliegenden Erfindung ist, zeigt nun das distale Ende 12a des Schlauches 12 für ein Vergleichsbeispiel der vorliegenden Erfindung näher. Zu sehen sind die beiden Lumen 22a und 22b des Katheterschlauches 12a, die sich längs desselben erstrecken, und, wie es in Fig. 4 gezeigt ist, unterschiedliche Querschnitte aufweisen. Alternativ können dieselben natürlich auch gleiche Querschnitte aufweisen. Wie es zu sehen ist, öffnet sich das Lumen 22b an der seitlichen Lumenöffnung 20b, das von der distalen Spitze 24 des Katheters etwas entfernt angeordnet ist, während sich das Lumen 22a an der Lumenöffnung 20a an der distalen Spitze 24 öffnet. Das Lumen 22b erstreckt sich aber über die Lumenöffnung 20b hinaus noch ein wenig in die Richtung der distalen Spitze 24, um an der distalen Spitze 24 an einem Teil 24a der Spitze 24 zu enden. Das Lumen 22b bildet somit von der Lumenöffnung 22b aus in Richtung der distalen Spitze 24 eine Sacköffnung, die optional an ihrem distalen Ende teilweise oder vollständig mit Material 26 gefüllt sein kann, wie es im Folgenden noch im Rahmen der Herstellungsbeschreibung beschrieben wird. Diese Sacköffnung bildet aber ferner einen Hohlraum 28, der an dem offenen Ende der Sacköffnung bzw. in der Nähe der Lumenöffnung 20b durch ein Abschlusselement 30 abgeschlossen ist. Das Abschlusselement 30 schließt den Hohlraum 28 in dem bezüglich der Lumenöffnung 20b distalen Teil des Lumens 22b fluiddicht ab und besteht beispielsweise aus dem gleichen Material wie das die Lumen 22a und 22b bildende Material 32. Der Hohlraum 28 ist beispielsweise mit Luft gefüllt, kann aber auch mit Vakuum oder einem aufgeschäumten oder porösen Material gefüllt sein.

Der Zwei-Lumen-Katheter gemäß Fig. 2 und 4, welcher nicht Bestandteil der vorliegenden Erfindung ist, bietet Vorteile bei der Verwendung als beispielsweise Venenkatheter. Um dies zu verdeutlichen, wird im Folgenden das Legen des Katheters beschrieben. Zunächst wird der Mehrlumentkatheter in den Patienten eingeführt, wie z.B. in eine Vene am Hals. Nachdem das distale Ende 12a in die Vene eingeführt worden ist, wird der Katheter 10 weiter entlang der Vene in Richtung des Herzens vorgeschoben, wobei dies mit oder ohne Verwendung eines Führungsdrahtes erfolgen kann. In dem Körper des Patienten wird der Zwei-Lumen-Katheter 10 mittels Ultraschall beobachtet. In den Ultraschallbildern ist zwar das Material 32 des Katheters aufgrund des geringen akustischen Impedanzunterschiedes zu dem umgebenden Blut nicht gut sichtbar, sehr wohl aber der in der Nähe der distalen Spitze 24 befindliche Hohlraum 28, der aufgrund seiner Grenzfläche von dem akustisch dicken Material 32 bzw. Material 32 mit hoher akustischer Impedanz zu dem Inneren des Hohlraumes 28, das akustisch dünn ist bzw. eine niedrigere Impedanz aufweist, die Ultraschallwellen eines Ultraschallwandlers gut reflektiert und somit in den Bildern zu einem hohen Kontrast führt. Abhängig von der Beobachtung mittels Ultraschall bzw. der Lage des Hohlraums 28 in den Ultraschallbildern kann dann der Katheter zu der gewünschten Position navigiert bzw. vorgeschoben werden, wie z.B. derart, dass die Spitze 24 unmittelbar vor dem Herzen positioniert wird. Vorteilhafterweise sind Ultraschallgeräte weniger aufwendig, groß und teuer sowie in ihrem Umgang weniger komplex als Röntgengeräte, so dass die Lagekontrolle bei Legen des Katheters gemäß Fig. 2 und 4 im Vergleich zu einer Röntgendurchleuchtungslösung vereinfacht ist.

Bevor bezugnehmend auf Fig. 3 ein weiteres Beispiel eines Zwei-Lumen-Katheters beschrieben wird, welches ebenfalls nicht Bestandteil der vorliegenden Erfindung ist, wird im Folgenden ein mögliches Herstellungsverfahren für den Katheter von Fig. 2 beschrieben. Zunächst wird ein Katheterschlauch mit den Lumen 22a und 22b aus dem Material 32 erzeugt, wie z.B. durch Extrudieren. Das Ergebnis ist ein Schlauch mit durchgehend konstantem Querschnitt, wie z.B. mit demjenigen von Fig. 4. Anschließend wird das Lumen 22b an dem distalen Ende 12a geschlossen, so dass aber weiterhin das Lumen 22a seine Lumenöffnung 20a an der Spitze des Katheterschlauches am dem distalen Ende 12a aufweist. Dazu wird beispielsweise das Lumen 22b an der distalen Schnittkante des extrudierten Schlauches mit einem Material 26 abgeschlossen, und eine Spitze 24 mit dem Abschlussteil 24a an die so vorbereitete Schnittfläche angefügt. Das Material 26 und das Material der Spitze 24 können jeweils zu dem Material 32 des Katheterschlauches gleich oder unterschiedlich sein. Das Material 32 ist beispielsweise Polyurethan mit oder ohne einem Anteil von Bariumsulfat. Das Abschließen des Lumens 22b kann auch über ein thermisches Verschließen und eine thermische und/oder mechanische Formung der Spitze 24 zu der in Fig. 2 gezeigten Form erfolgen. Vor oder nach Bildung der Spitze 24 bzw. distalseitigem Abschluss des Lumens 22b wird die seitliche Lumenöffnung 20b gebildet, wie z.B. durch einen in Querrichtung stattfindenden Schneidevorgang. Anschließend wird das Lumen 22b mittels des Abschlusselementes 30 verschlossen, wobei als Abschlussmaterial 30 beispielsweise Kleber verwendet wird, der durch die Öffnung 20b an die gewünschte Position gebracht wird. Alternativ kann für das Element 30 das gleiche Material wie das Material 32 oder Material 26 verwendet werden, das geeignet mit dem Material 32 verbunden wird, wie z.B. mittels eines Lösungsmittels oder eines Haftmittels. In dem Hohlraum 28 befindet sich dann die Atmosphäre, die bei Abschließen des Hohlraumes mit dem Element 30 vorlag, wie z.B. Luft.

Alternativ ist es natürlich ebenfalls möglich, das Abschlusselement 30 vor dem Abschließen des Lumens 22b bzw. der Bildung der distalen Spitze 24 von der distalen Seite aus durch das Lumen 22b bzw. den in diesem Augenblick noch an dem distalen Ende offenen Hohlraum 28 in das Lumen 22b einzubringen und den Hohlraum 28 dann schließlich erst später bei Bildung der Spitze 24 abzuschließen. Auch könnte die Bildung der Lumenöffnung 20b in einer anderen Reihenfolge bezüglich der übrigen Schritte stattfinden. Ferner könnte vor dem Abschließen des Hohlraumes 28 durch das Abschlusselement 30 bzw. den Teil 24a der Spitze 24 derselbe mit einem aufgeschäumten oder porösen Material 34 gefüllt werden. Aufgrund seiner Luft- bzw. Gaseinschlüsse führt dieses Material ebenfalls zu einem guten Kontrast in Ultraschallbildern. Dieses aufgeschäumte Material wäre dann durch das Abschlusselement 30 bzw. das Material 32 gegenüber dem umgebenden Serum, wie z.B. Blut, getrennt, so dass dessen eventuell zerklüftete Oberfläche keinen Angriffspunkt für Biobewuchs bzw. die Anlagerung von Keimen bietet, und die Oberfläche des Katheters weiterhin durchweg glatt sein kann, um Anlagerungen solcher Keime zu verhindern. Alternativ kann das Abschlussmaterial 30 weggelassen und das poröse Material 34 mit einer glatten Grenzfläche hergestellt werden, wofür ein Beispiel im Folgenden bezugnehmend auf Fig. 3 erläutert wird.

Fig. 3, nicht Bestandteil der vorliegenden Erfindung, zeigt ein zu Fig. 2 alternatives Beispiel für ein distales Ende 12a' des Zwei-Lumen-Katheters von Fig. 1, wobei jedoch der Querschnitt A-A aus Fig. 4 auch auf dieses Beispiel zutrifft. Das Vergleichsbeispiel von Fig. 3 unterscheidet sich von demjenigen von Fig. 2 dadurch, dass der Abschnitt des Lumens 22b mit der seitlichen Lumenöffnung 20b, der sich von der Öffnung 20b bis zu dem distalen Ende dieses Lumens 22b erstreckt, nicht etwa zu einem Hohlraum abgeschlossen ist, sondern lediglich mit aufgeschäumten Material 34 aufgefüllt ist, das Lufteinschlüsse aufweist, die wiederum die oben skizzierten Vorteile bei der Ultraschallbildverarbeitung haben. Als aufgeschäumtes Material 34 kann beispielsweise Polyurethan mit und ohne geeigneten Zusätzen verwendet werden. Ähnlich wie im vorhergehenden bezüglich des Abschlusselementes 30 beschrieben, kann das aufgeschäumte Material 34 bei Herstellung des Katheters von Fig. 3 durch die seitliche Lumenöffnung 20b irgendwann nach Bilden der Öffnung 20b und vorzugsweise auch der Anfügung der Spitze oder durch eine distale Öffnung des Lumens 22b zu einem Zeitpunkt vorgenommen werden, bei dem die Spitze 24 mit dem Abschlussteil 24a noch nicht angefügt bzw. gebildet worden ist. Bezüglich der Vorteile des Katheters von Fig. 3 beim Legen desselben, wird auf die Ausführungen bezüglich des Katheters von Fig. 2 verwiesen, die diesbezüglich auf das Vergleichsbeispiel ebenfalls zutreffen. Im Hinblick auf die Herstellung des Katheters von Fig. 3 treffen die obigen Ausführungen bezüglich Fig. 2 zu, soweit sie nicht bezugnehmend auf Fig. 3 abweichend beschrieben wurden.

Bei dem Katheter von Fig. 3 liegt zwar ein Teil der rauen Oberfläche des aufgeschäumten Materials (34) gegenüber dem umgebenden Serum, wie z.B. Blut, frei, aber da die Zone örtlich sehr beschränkt ist, ist die Gefahr durch Keimverunreinigungen bzw. -Anlagerungen nur gering. Schließlich kann auch das Material 34 mit einer glatten Grenzfläche 34a hergestellt werden.

Abschließend sei erwähnt, dass die obigen Vergleichsbeispiele lediglich exemplarisch auf einen Zwei-Lumen-Katheter eingingen, und dass dieselben ferner auch auf Mehrlumenkatheter mit mehr Lumen anwendbar sind. Allgemein kann bei einem n-Lumen-Katheter mit n-1 seitlichen Lumenöffnungen bzw. Seitenaugen der Abschnitt jedes dieser n-1 Lumen, der sich zwischen dem jeweiligen Seitenauge und dem zu dem distalen Ende des Mehrlumenkatheters weisenden Ende des Lumens befindet, verwendet werden, um einen abgeschlossenen Hohlraum 28 zu bilden bzw. aufgeschäumtes Material 34 aufzunehmen. Ferner wird darauf hingewiesen, dass auch die in Fig. 4 sichtbaren Querschnitte lediglich exemplarischer Natur sind. So kann ferner der äußere Umfang des Querschnittes des Katheterschlauches 12 auch anders geformt sein als kreisförmig, wie es in Fig. 4 gezeigt ist. Wie im vorhergehendem erwähnt, kann ferner das Material 26 am Ende des Lumens 22b fehlen.

Im Vorhergehenden wurde beschrieben, dass das Material 34 Gasblasen besitzt. Andere Materialien sind aber ebenfalls möglich, nämlich solche, die mit Ultraschall sichtbaren Stoffen vermischt sind. Das gilt sowohl für das Material 34 in Fig. 3 als auch für die "Füllung" des Hohlraums 28 in Fig. 2. Dabei soll in der vorliegenden Anmeldung beispielsweise etwas als in dem Körper eines Lebewesens mittels Ultraschall erkennbar bzw. sichtbar gelten, wenn es Ultraschall aktiv aussendet oder durch das Körperinnere auftreffenden Ultraschall mit einem ausreichenden Reflexionskoeffizienten reflektiert, wie z.B. durch Verwendung eines Materials im Inneren des Katheters mit einer akustischen Impedanz, die beispielsweise weniger als 10% der akustischen Impedanz von Wasser oder weniger als 10% der akustischen Impedanz des Kathetermaterials beträgt.

Zudem sind die obigen Vergleichsbeispiele dahingehend modifizierbar, dass anstelle des Vorsehens einer "passiven" Ultraschallmarkierung in Form des Hohlraums 28 bzw. des Materials 34 einer aktive Ultraschallmarkierungseinrichtung an dem distalen Ende des Mehrlumenkatheters vorgesehen wird oder zumindest in der Nähe der Lumenöffnungen. Beispielsweise kann in dem Teil des Lumens 22b zwischen der Lumenöffnung 20b und dem proximalen Ende desselben eine aktive "Sonde" in Form eines ultraschallaussendenden Senders eingeschoben sein. Vorzugsweise besitzt diese aktive Sonde eine eigene Energieversorgung in Form von beispielsweise einer Batterie. Die aktive Sonde könnte lediglich in das Lumen 22b gesteckt sein, und zwar vorzugsweise nur bis in die Nähe des Seitenauges 20b oder aber sogar bis in die Sacköffnung des Lumens 22b.

Anstatt einer aktiven Sonde, die Ultraschallsignale erzeugt und aussendet, könnte allerdings in das Lumen 22b auch eine "passive" Sonde in Form beispielsweise einer gasgefüllten Kugel bzw. eines gasgefüllten vorne und hinten abgeschlossenen Schlauches angeordnet, eingefügt bzw. befestigt sein, wie z.B. bis vor die Öffnung 22b oder bis in den Hohlraum 28 bzw. in die Sacköffnung aus Fig. 2 bzw. aus Fig. 3.

Die Einfügung der passiven oder aktiven Sonde bzw. die Herstellung eines Mehrlumenkatheters mit einer solchen kann auf die vorbeschriebene Weise erfolgen, indem bei der Herstellung nach Fig. 2 beispielsweise die Sonde in den Hohlraum 28 eingefügt wird, bevor derselbe durch das Material 30 oder die distale Spitze 24 abgeschlossen wird, oder indem bei der Herstellung nach Fig. 3 der Materialeinbringungsschritt für das Material 34 durch einen Sondeneinbringungsschritt ersetzt wird. Die Voreile, die sich durch die soeben beschriebenen modifizierten Vergleichsbeispiele mit aktiver und passiver Sonde im Zusammenhang mit dem Legen des Katheters ergeben, erschließen sich ohne weiteres aus der Beschreibung der Beispiele von Fig. 2 und 3, die in dieser Hinsicht auch auf die Modifizierungen zutrifft, wenngleich bei der Verwendung einer aktiven Sonde der Vorteil nicht in der Ausnutzung des hohen Reflexionsvermögens an der Grenzfläche von akustisch dickem zu akustisch dünnem Material liegt, sondern in der Ausnutzung des direkt durch die aktive Sonde ausgestrahlten Ultraschallsignals. Eine geeignete aktive Sonde kann eine Einrichtung zur Aktivierung und ggf. Deaktivierung der Ultraschallsignalerzeugung abhängig von einem Auslöseereignis aufweisen, wie z.B. abhängig von einem elektromagnetischen Auslösesignal, so dass die Ultraschallsignalerzeugung auf den Zeitraum des Einsatzes des Katheters beschränkt werden kann.

Die vorhergehenden Vergleichsbeispiele beschäftigten sich mit Kathetern, bei denen die Ultraschallmarkierungseinrichtung in Form des Hohlraums 28, des Materials 34 bzw. der passiven/aktiven Sonde für einen dauerhaften Verbleib in dem Mehrlumenkatheter und insbesondere in dem ansonsten ungenutzten Teil eines Lumens mit Seitenauge vorgesehen war, wenngleich in dem Fall der lediglich eingesetzten passiven/aktiven Sonde selbige grundsätzlich über das Lumen 22b entfernbar wäre. Bei dem folgenden Ausführungsbeispiel wird das Legen eines Katheters zielgenau zu einer Sollposition, wie z.B. kurz vor dem Herz, dadurch erleichtert, dass ein Lumen eines Katheters, der bei diesem Ausführungsbeispiel auch ein Einlumenkatheter sein kann, vorübergehend mit einer wieder durch das Lumen entfernbaren passiven oder aktiven Sonde versehen wird. Ein solches Ausführungsbeispiel wird bezugnehmend auf die Figuren 5 und 6 näher erläutert, die das distale Ende 50 bzw. das proximale Ende 52 eines Katheters 54 zeigen, an dessen proximalem Ende 52 ein Anschluss 56, wie zum Beispiel ein Luerlockanschluss, angeordnet ist. Gemäß dem Ausführungsbeispiel von Fig. 5 und 6 ist der Katheter 54 ein Einlumenkatheter mit einem Lumen 58 und bildet zusammen mit einer ebenfalls gezeigten passiven Sonde 60 ein Kathetersystem, das das Legen des Katheters 54 wie im Folgenden beschrieben erleichtert. Dafür ist die passive Sonde 60 hier als ein flexibler Schlauch ausgebildet, dessen Außenumfang seines Querschnitts klein genug ist, so dass der Schlauch 60 in das Lumen 58 über den Anschluss 56 bzw. das proximale Ende 52 eingeführt werden kann. Insbesondere ist dabei der Schlauch 60 lang genug, um sich von dem distalen Ende 50 durch das Lumen 58 über den Anschluss 56 hinausragend nach außen zu erstrecken. Genauer ausgedrückt ist der Schlauch 60 lang genug, um sich von einer Nähe einer Lumenöffnung 62 des Lumen 60 an einer Lumenspitze 64 des distalen Endes 50 durch das Lumen 58 über den Anschluss 56 hinaus zu erstrecken, wobei für einen Betreiber eine Markierung 66 sichtbar an dem Äußeren des Schlauches 60 vorgesehen ist, um in einer bis in die Nähe der Spitze 62 eingefügten Stellung des Schlauches 60 sich in einer vorbestimmten Entfernung zu dem Anschluss 56 zu befinden, wie z.B. unmittelbar davor. Der Schlauch 60 ist im Inneren hohl und zudem an seinem distalen Ende 60a und beispielsweise auch seinem proximalen Ende 60b verschlossen, um in Inneren einen beispielsweise gasgefüllten Raum 68 zu bilden.

Nachdem im Vorhergehenden die Komponenten des Kathetersystems von Fig. 5 und 6 beschrieben worden sind, wird im Folgenden dessen Einsatz beim Legen des Katheters 54 beschrieben. Vor dem Einsatz bzw. vor dem Legen des Katheters 54 oder während des Einsetzens bzw. des Legens befinden sich Katheter 54 und flexibler Schlauch 60 in dem in den Fig. 5 und 6 gezeigten Zustand, nämlich in dem Zustand, dass der Schlauch 60 mit seinem distalen Ende 60a bis in die Nähe der distalen Spitze 62 des Katheters 54 in das Lumen 58 eingeführt ist, und zwar beispielsweise steril verpackt. Sobald der Katheter 54 beispielsweise in eine Vene eingeführt bzw. katheterisiert worden ist, wird er die Vene entlang in die Nähe einer Sollposition, wie z.B. vor dem Herzen, vorgeschoben. Da sich der Schlauch 60 als passive Sonde in dem Lumeninneren 58 befindet und sich bis in die Nähe der Lumenspitze 62 erstreckt, kann wie im Vorhergehenden beschrieben mittels Ultraschall die Lage der Katheterspitze 62 bzw. des gesamten Katheters 54 relativ zu der Sollposition kontrolliert und damit das Navigieren des Katheters 54 mit seiner Spitze 62 an die Sollposition gesteuert werden, da sich durch die Grenzfläche von Schlauchmaterial zu Schlauchinnerem 68 in den Ultraschallbildern ein guter Kontrast der passiven Sonde 60 ergibt. Anhand der Markierung 66 kann der Arzt dabei sicherstellen, dass sich das distale Ende 60a in der gewünschten Nähe zu der Katheterspitze 62 befindet. Der verbleibende Versatz, wie er in Fig. 5 zu sehen ist, ist beispielsweise dem Betreiber im Vorhinein bekannt, so dass er denselben bei der Beobachtung der Ultraschallbilder bei der Lagekontrolle berücksichtigen kann. Nachdem auf diese Weise die Katheterspitze 62 wie gewünscht an die Sollposition herangeführt worden ist, kann die passive Sonde 60 durch das Lumen 58 aus demselben entfernt werden, wozu der Schlauch 60 an seinem überstehenden proximalen Ende gezogen werden kann. Die passive Sonde 60 ist dabei beispielsweise als ein Einwegartikel vorgesehen.

Zur Vorbereitung des Legens des Katheters 54 ist also der Schlauch 60 in das Lumen 58 bis zu dem distalen Ende 50 eingeführt worden, um dann beispielsweise verpackt und zusammen mit dem Katheter 54 sterilisiert zu werden, wie z.B. durch eine sterilisationsgaspermeable Verpackung.

Bezugnehmend auf das vorhergehende Ausführungsbeispiel von Fig. 5 und 6 wird darauf hingewiesen, dass dasselbe nicht auf ein Einlumenkatheter beschränkt ist. Vielmehr kann eine passive Sonde 60 natürlich auch bei einem Mehrlumenkatheter eingesetzt werden, wobei die Einführung in jedes der Lumen stattfinden kann, wie z.B. aber nicht ausschließlich das Lumen mit seiner Lumenöffnung an der Katheterspitze. Ferner wird darauf hingewiesen, dass statt der beschriebenen passiven Sonde auch eine längliche aktive Sonde verwendet werden könnte, die beispielsweise ein ähnliches Äußeres aufweist, wie der vorbeschriebene flexible Schlauch 60, die aber an ihrem distalen Ende einen Ultraschallsender aufweist. Schließlich kann als passive Sonde auch ein massiver flexibler Stab verwendet werden, solange er die Ultraschallsichtbarkeit ermöglicht oder ein flexibler Stab aus aufgeschäumten Material oder dergleichen.

Obwohl im Vorhergehenden beschrieben worden ist, dass die passive Sonde 60 beispielsweise zusammen mit dem Katheter 54 in einem eingeführten Zustand verpackt ist, kann es auch vorgesehen sein, dass beide Bestandteile des Kathetersystems, nämlich Katheter 54 und-passive Sonde 60 getrennt verpackt sind. Beim Legen des Katheters 54 kann dieser zunächst in beispielsweise eine Vene eingeführt und ein wenig vorgeschoben werden, um daraufhin die passive Sonde 60 in das Lumen 58 einzuführen. Entweder kann durch dasselbe die passive Sonde 60 durch die Vene zu der Sollposition vorgeschoben werden, um erst bei mittels ultraschallkontrolliertem Erreichen des distalen Endes 60a der passiven Sonde der Sollposition den Katheter 54 bis zu der Markierung 66 nachzuschieben, und daraufhin die passive Sonde 60 aus dem Lumen 58 wie im Vorhergehenden beschrieben zu entfernen, oder der Katheter wird mit Hilfe der Sonde bis zu seiner Sollposition über eine Ultraschallkontrolle navigiert.

Schließlich soll noch darauf hingewiesen werden, dass es möglich ist, beispielsweise in die bestehende Software eines Ultraschallgerätes eine als Vorrichtung zur Anzeige eines Signals zur Katheternavigation wirkende Softwareroutine zu integrieren, die in der Lage ist, in den Ultraschallbildern der Ultraschallbildsequenz, die erzeugt wird, während einer der vorbeschriebenen Katheter die Vene entlang in Richtung des Herzens geschoben wird, die Ultraschallmarkierungseinrichtung, wie z.B. den abgeschlossenen Hohlraum bzw. die aufgeschäumte Materialfüllung, oder die passive/aktive Sonde, zu erkennen und auch die Lage desselben bzw. derselben zu erkenne, und basierend auf dieser erkannten Lage ein Signal für beispielsweise den Arzt anzuzeigen, dass für eine Navigation des Mehrlumenkatheters zu der gewünschten Position vor dem Herzen dient. Beispielsweise ertönt ein akustisches Geräusch in dem Augenblick, an dem die Katheterspitze an dem gewünschten Ort vorbeigleitet, wobei die Softwareroutine hierzu beispielsweise auch den longitudinalen Versatz zwischen der Ultraschallmarkierungseinrichtung, wie z.B. dem Hohlraum (28) bzw. dem aufgeschäumten Material (34) oder der aktiven/passiven Sonde, der bzw. das in den Ultraschallbildern beobachtet wird, zu der Spitze (24) berücksichtigt. Natürlich kann die soeben beschriebene Navigationssignalanzeigeroutine auch in Firmware oder Hardware implementiert sein. Zudem kann die Routine auch automatisch die Soll-Position erkennen, beispielsweise indem der Routine gegenüber durch den Benutzer lediglich angezeigt wird, dass "das distale Ende vor dem venösen Eingang des Herzens" angeordnet sein soll bzw. ein "zentraler Venenkatheter" erwünscht ist, woraufhin die Routine diesen Ort an bestimmten Merkmalen automatisch in den Ultraschallbildern erkennt.

Schließlich wird abschließend noch darauf hingewiesen, dass die vorliegende Erfindung nicht auf Venenkatheter beschränkt ist. Vielmehr ist die vorliegenden Erfindung auch auf andere Katheter anwendbar und auf andere Kathetereinsätze in anderen Seren als Blut.

## Patentansprüche

1. Katheter mit einer in einem Körper eines Lebewesens mittels Ultraschall erkennbaren Ultraschallmarkierungseinrichtung, wobei der Katheter ein Lumen (58) aufweist, und die Ultraschallmarkierungseinrichtung länglich und so über ein an einem proximalen Ende (52) des Katheters angeordneten Anschluss (56) in das Lumen (58) einführbar ist, dass sich dieselbe von der Nähe eines distalen Endes des Katheters aus durch das Lumen (58) über das proximale Ende (52) des Katheters hinaus bis zu dem Anschluss (56) des Katheters erstreckt, um von dem Anschluss (56) vorzustehen, und dass dieselbe über das proximale Ende (52) des Katheters entfernbar ist, **dadurch gekennzeichnet, dass** die Ultraschallmarkierungseinrichtung ein an dessen distalem Ende (60a) geschlossener Schlauch (60) ist.

2. Katheter gemäß Anspruch 1, bei dem die Ultraschallmarkierungseinrichtung eine Einrichtung zum Erzeugen und Ausgeben eines Ultraschallsignals aufweist.

3. Katheter gemäß Anspruch 2, wobei der Katheter als ein Katheter ausgebildet ist, der ein erstes Lumen (22a) mit einer Lumenöffnung (20a) an einer Spitze des Katheters an dem distalen Ende (12a) und ein zweites Lumen (22b) mit einer seitlichen Lumenöffnung (20b) aufweist, die proximaler angeordnet ist als die erste Lumenöffnung, wobei die Ultraschallmarkierungseinrichtung in einem Teil des zweiten Lumens (22b) anordbar ist, der sich zwischen der ersten Lumenöffnung (20b) und der zweiten Lumenöffnung (20a) befindet.

4. Katheter gemäß einem der vorhergehenden Ansprüche, der als zentraler Venenkatheter geeignet ist.

5. Verfahren zum Vorbereiten eines Katheters für ein Legen desselben, mit folgendem Schritt:
Einfügen einer Ultraschallmarkierungseinrichtung, die in einem Körper eines Lebewesens mittels Ultraschall erkennbar und länglich ist, um sich von der Nähe eines distalen Endes des Katheters aus durch das Lumen (58) über ein proximales Ende (52) des Katheters hinaus bis zu einem Anschluss (56) des Katheters erstrecken zu können, um von dem Anschluss (56) vorzustehen, in das Lumen (58) bis zu der Nähe des distalen Endes (50), **dadurch gekennzeichnet, dass** die Ultraschallmarkierungseinrichtung ein an dessen distalem Ende (60a) geschlossener Schlauch (60) ist.

6. Verfahren gemäß Anspruch 5, der ferner folgenden Schritt aufweist:
Verpacken des Katheters mit der eingefügten Ultraschallmarkierungseinrichtung in einer Verpackung.

## Claims

1. Catheter with an ultrasound marking means, which is recognizable by means of ultrasound in the body of a living being, wherein the catheter comprises a lumen (58), and the ultrasound marking means is elongated and insertable into the lumen (58) via a connection (56) arranged at a proximal end (52) of the catheter such that said ultrasound marking means extends from near a distal end of the catheter through the lumen (58) beyond the proximal end (52) of the catheter to the connection (56) of the catheter so as to project from the connection (56), and that said ultrasound marking means is removable via the proximal end (52) of the catheter, **characterized in that** said ultrasound marking means is a tube (60) closed at its distal end (60a).

2. Catheter according to claim 1, wherein the ultrasound marking means comprises means for generating and outputting an ultrasound signal.

3. Catheter according to claim 2, wherein the catheter is formed as a catheter comprising a first lumen (22a) with a lumen opening (20a) at a tip of the catheter at the distal end (12a), and a second lumen (22b) with a lateral lumen opening (20b) arranged more proximally than the first lumen opening, wherein the ultrasound marking means may be arranged in a part of the second lumen (22b) located between the first lumen opening (20b) and the second lumen opening (20a).

4. Catheter according to one of the preceding claims, which is suitable as a central venous catheter.

5. Method of preparing a catheter for introducing the same, comprising:
inserting an ultrasound marking means, which is recognizable in the body of a living being by means of ultrasound and is elongated so as to be able to extend from near a distal end of the catheter through the lumen (58) beyond a proximal end (52) of the catheter to a connection (56) of the catheter so as to project from the connection (56), into the lumen (58) to the proximity of the distal end (50), **characterized in that** said ultrasound marking means is a tube (60) closed at its distal end (60a).

6. Method according to claim 5, further comprising:
packaging the catheter, with the ultrasound marking means inserted, in a package.

## Revendications

1. Cathéter avec un moyen de marquage ultrasonore identifiable par ultrasons dans un corps d'un être vivant, le cathéter présentant une lumière (58), et le moyen de marquage ultrasonore pouvant être introduit longitudinalement dans la lumière (58) via une connexion (56) disposée à une extrémité proximale (52) du cathéter de sorte qu'il s'étende de la proximité de l'extrémité distale du cathéter, à travers la lumière (58), au-delà de l'extrémité proximale (52) du cathéter jusqu'à la connexion (56) du cathéter, pour faire saillie de la connexion (56), et qu'il puisse être éloigné via l'extrémité proximale (52) du cathéter, **caractérisé par le fait que** le moyen de marquage ultrasonore est un tube flexible (60) fermé à son extrémité distale (60a).

2. Cathéter selon la revendication 1, dans lequel le moyen de marquage ultrasonore présente un moyen pour générer et sortir un signal ultrasonore.

3. Cathéter selon la revendication 2, dans lequel le cathéter se présente sous forme de cathéter présentant une première lumière (22a) avec une ouverture de lumière (20a) à une pointe du cathéter à l'extrémité distale (12a) et une deuxième lumière (22b) avec une ouverture de lumière latérale (20b) disposée plus proximale que la première ouverture de lumière, le moyen de marquage ultrasonore pouvant être disposé dans une partie de la deuxième lumière (22b) se trouvant entre la première ouverture de lumière (20b) et la deuxième ouverture de lumière (20a).

4. Cathéter selon l'une des revendications précédentes, convenant comme cathéter pour veine centrale.

5. Procédé pour préparer un cathéter pour un placement de celui-ci, à l'étape suivante:
introduire un moyen de marquage ultrasonore, qui est identifiable par ultrasons dans un corps d'un être vivant et qui est allongé, pour pouvoir s'étendre de la proximité d'une extrémité distale du cathéter, à travers la lumière (58), au-delà de l'extrémité proximale (52) du cathéter jusqu'à une connexion (56) du cathéter, pour faire saillie de la connexion (56), dans la lumière (58) jusqu'à proximité de l'extrémité distale (50), **caractérisé par le fait que** le moyen de marquage ultrasonore est un tube flexible (60) fermé à son extrémité distale (60a).

6. Procédé selon la revendication 5, présentant par ailleurs l'étape suivante:
emballer le cathéter avec le moyen de marquage ultrasonore introduit dans un emballage.
